# EUROPEAN PATENT SPECIFICATION

(11) **EP 0 454 383 B1**
(45) Date of publication and mention of the grant of the patent: **28.06.1995**
(21) Application number: 91303560.6
(22) Date of filing: 22.04.1991
(51) Int. Cl.: A61K 9/50, A61K 9/48, A61K 9/52

(54) **Large intestinal dissociative polypeptide series oral formulation**
Im Dickdarm zerfallende, Polypeptide enthaltende orale Formulierungen
Formulation orale contenant des polypeptides dissociables dans le gros intestin

(30) Priority: 23.04.1990 JP 107060/90; 05.04.1991 JP 100304/90
(43) Date of publication of application: 30.10.1991
(73) Proprietor: AICELLO CHEMICAL CO., LTD., Toyohashi-shi, Aichi-ken 441-11 (JP)
(72) Inventor: Fujii, Takeru, Naruto-shi, Tokushima-ken (JP); Takama, Shigeyuki, Ohkawa-gun, Kagawa-ken (JP); Watoh, Takahiko, Ohkawa-gun, Kagawa-ken (JP); Suzuki, Tsutomu, Toyohashi-shi, Aichi-ken (JP); Matsumoto, Takayuki, Toyohashi-shi, Aichi-ken (JP)
(74) Representative: Harvey, David Gareth

(56) References cited:
- WO-A-89/01034

## Description

The present invention relates to a oral medicine for dissociating in the large intestine an oral administrating formulation containing a physiologically active polypeptide (hereinafter, is referred to simply as polypeptide). The medicine is contained in capsules of the kind known as "hard capsules".

Physiologically active polypeptide hormones such as insulin, calcitonin, etc., are water-soluble high-molecular compounds which are easily decomposed with an intestinal protease such as pepsine, trypsin, etc. Accordingly, when these polypeptides are orally administered, they are decomposed with the above-described protease and are scarcely adsorbed from the mucous membrane of an alimentary canal, whereby the effective medical action of the polypeptides can not be expected.

Thus, for obtaining the physiological activity of the polypeptide, it is prepared as in injection only. However, when the polypeptide must be periodically and frequently administered, the administration by injection gives large pain to a patient and hence recently, the development of other administrating methods of the above-described polypeptide than injection has been investigated with great effort. Hitherto, various polypeptide formulations for a nasal cavity administration or a vaginal administration have been developed and various kinds of additives showing the absorption acceleration action of polypeptide have been proposed. However, it can not be denied that the above-described administration methods are inconvenient for practical use and hence it has been desired that the method should involve an oral administration.

Accordingly, various investigations for making the polypeptide, which is reluctant to be adsorbed from the lower alimentary canal, in an easily absorbable form have been made.

However, many of the oral formulations which have hitherto been proposed are estimated to be absorbed in the duodenum. In the polypeptide oral medicine prepared by such a manner, recently, various intestinal coating agents which are insoluble in the gastric juice have been developed and the occurrence of the hydrolysis of a polypeptide in a stomach can be avoided for the present but since these coating agents are dissolved in the duodenum, the polypeptide is decomposed by protease in the duodenum and hence a method of effectively absorbing a polypeptide in the large intestine after the duodenum has not yet been discovered.

WO-A-8901034 discloses microcapsules obtained by suspending a pharmaceutical compound in a medium comprisisng chitosan as a gelling inducer, forming droplets from the suspension and contacting them with a gelling solution to form microcapsules having semi-permeable capsule walls.

The object of the present invention is to provide an oral medicine in the form of a hard capsule, which is dissociative in the large intestine so that a physiologically active peptide can be effectively absorbed in the large intestine.

As the result of investigations on the optimum absorption site under the consideration that if the absorption site for a polypeptide is the large intestine after the duodenum, the polypeptide is reluctant to be influenced by protease, the inventors have confirmed that the optimum site is the large intestine after the duodenum, in particular the colon. Also, as the result of making further various investigations on a formulation which is dissociated after reaching the colon based on the above result, it has been discovered that since chitosan or a composite of chitosan and a powdery cellulose and/or starch are not dissolved in an alkali solution and are degraded by intestinal microorganisms, by using chitosan or the composite thereof as a protective agent for a polypeptide medicament and by coating a polypeptide medicament using a polymer, which is soluble in a solution having pH of at least 5 but is insoluble in the gastric juice having pH of 4 or lower, as an external protective agent for protecting from the gastric juice, the above-described object can be effectively achieved, the inventors have succeeded in accomplishing this invention based on the discovery.

That is, it has now been discovered that the above-described object can be achieved by the present invention as described hereinbelow.

Thus, according to this invention, there is provided an oral medicine which is dissociative in the large intestine, comprising a physiologically active polypeptide formulation encapsulated within a molded chitosan hard capsule, the capsule having a surface coated by a layer of a polymer soluble in a liquid of pH5 or higher.

The invention also provides an oral medicine which is dissociative in the large intestine comprising a physiologically active polypeptide formulation encapsulated within a hard capsule composed of chitosan and a powdery cellulose or starch, or a mixture of such cellulose and starch, the capsule having a surface coated by a layer of a polymer soluble in a liquid of pH5 or higher.

The invention will now be described in more detail in the following description.

Chitosan for use in this invention is a material obtained by treating chitin made from Crustacean such as a lobster, a crab, etc., with a concentrated alkali to completely or partially deacetilate the acetyl group. In this invention, it is preferable to use chitosan having 100 mPa.s (100 cps) or lower at 20°C in the viscosity of the solution formed by dissolving the chitosan in an aqueous solution of 1% by weight acetic acid at a concentration of 1% by weight, and having at least 60 mol% in the deacetilation degree. If chitosan having the viscosity of the solution of 1% by weight over 100 mPa.s (100 c.p.s.) and the deacetilation degree of less than 60 mol% is used, the solubility thereof in an acetic solution is greatly reduced and hence the concentration of the solution thereof which is used for forming the the capsule can not be increased, whereby the formation of the capsule becomes very difficult. Furthermore, in any case since the solubility of the chitosan at the pH of the body fluid in the large intestine and the degradation thereof with microorganisms or enzymes existing in the large intestine are low, the dissociation of the formed medicine capable of releasing the medicament is not attained. On the other hand, if the deacetylation degree is over 98 mol%, the crystallinity of the chitosan is increased and in this case, the dissociating property in the large intenstine is greatly reduced by the same reason as described above.

In addition, chitosan derivatives such as carboxymethylated chitosan, hydroxymethylated chitosan, phosphoric acid esterified chitosan, sulfuric esterified chitosan, etc., each having a low substitution of less than few mol% are included in chitosan for use in this invention.

Furthermore, in this invention, a powdery cellulose and/or starch may be mixed with or dispersed in chitosan to accelerate the dissociation of the medicine with the microorganisms in the large intestine.

As the powdery cellulose for use in this invention, one can use a powdery cellulose passing a sieve of 149 »m (100 mesh), and preferably passing a sieve finer than 74 »m (200 mesh), and obtained by mechanically or chemically pulverizing a natural cellulose. Since the powdery cellulose is suspended in a chitosan solution, the viscosity is scarcely increased, the concentration of the chitosan solution can be increased and also the powdered cellulose has an effect of improving the strength of the formed medicament and accelerating the degradation thereof with microorganisms in the large intestine. Also, fine crystal cellulose obtained by refining a natural cellulose after decomposing non-crystalline parts from the natural cellulose can be also used in this invention, but is inferior to the aforesaid cellulose from the standpoint of economy.

Furthermore, in this invention, starch may be used in place of cellulose or together with cellulose. When starch is used, by mixing with or dispersing in chitosan, the degradation of the medicament with microorganisms in the large intestine can be accelerated is in the case of using the powdery cellulose. Examples of the starch for use in this invention are rice starch, corn starch, and potato starch.

As the polymer soluble in a solution having pH of at least 5 for coating the surface according to the present invention, one can use a polymer which is conventionally used as intestinal capsules or for coating tablets for the purpose of importing gastric juice resistance to the formed medicament.

Examples of such a polymer are anionic acryl resins such as a methacrylic acid-methyl methacrylate copolymer, a methacrylic acid-ethyl acrylate copolymer [e.g., Eudragit S L (registered trade name), made by Roehm Pharm Co.], etc., hydroxypropylmethyl cellulose acetate succinate, and hydroxypropylmethyl cellulose phthalate.

The polypeptide being contained in the polypeptide oral medicine can include any polypeptide but is preferably a polypeptide having a relatively low molecular weight.

Examples of such a polypeptide are insulin, angiotensin, vasopressin, desmopressin, LH-RH (luteinizing hormone), somatostatin, calcitonin, glucagon, oxytocin, gastorin, somatomedin, secretin, h-ANP (human atrium sodium urination peptide), ACTH (adrenocorticotropic hormone), MSH (melanocyte stimulating hormone), β-endorphin, muramyl-dipeptide, enkephalin, neuro tensin, bombesin, VIP (vasoactive intestinal polypeptide), CCK-8 (cholecystokinin-8), PTH (pirathyroid hormone), CGRP (calcitonic gene related peptide), TRH (thyrotropin-releasing hormone), endocerine, and the derivatives of them.

The above-described polypeptides include not only natural polypeptides but also pharmacologically active derivatives thereof and analogs of them. Accordingly, calcitonin for use in this invention includes not only naturally existing materials such as salmon calcitonin, human calcitonin, pig calcitonin, eel calcitonin, chicken calcitonin, etc., but also analogs thereof such as [Asul, 7]-eel calcitonin (erucatonic), etc.

It is preferable that the oral medicine of this invention contains, if necessary, in absorption accelerator and as such an absorption accelerator, a combination of a nonionic surface active agent and a middle chain aliphatic carboxylic acid or an alkali metal salt thereof is particularly preferred.

At the preparation of the polypeptide oral medicine of this invention, a polypeptide is dissolved in purified water as a carrier or a diluent, or an isotonic sodium chloride solution or a buffer solution and after solidifying the solution by adding thereto a binder, etc., the solidified product is formed into a desired form. In this case, as the form of the formulation, there are capsules, tablets, pills, granules, fine grains, powders, etc.

At the preparation of the formulation of the present invention, if necessary, an animal or vegetable protein may be contained in the formulation. The above-described protein is not an inevitable component in this invention but when the count of the polypeptide or a derivative thereof is fine, the protein acts as a stabilizer for the polypeptide.

The pH of the formulation of this invention is adjusted as follows. That is, after or before dissolving a physiologically active polypeptide and, if necessary, an absorption accelerator, an animal protein, a vegetable protein, etc., in purified water, there is added an isotonic sodium chloride solution, or a buffer solution, the pH of the solution is adjusted to the range of from 3 to 7, and preferably from 3 to 7. The compound which is used for adjusting pH may be a known acid or base having no toxicity to man and causing no simulation and preferred examples thereof are organic acids such as acetic acid, citric acid, etc., and weak bases such as sodium hydrogencarbonate, sodium acetate, etc.

For preparing the capsules, a polypeptide medicament is encapsulated with capsules prepared by a material containing chitosan or a mixture of chitosan and a powdery cellulose and/or starch and then coated with a polymer soluble in a liquid having pH of at least 5.

Also, for preparing medicaments according to this invention, a liquid composition containing a polypeptide medicament and chitosan or a material containing chitosan and a powdery cellulose and/or starch are sufficiently mixed with suitable additives such is an excipient, a binder, etc., followed by drying, after adding, if necessary, other additives such as a lubricant, etc., the final mixture can be pulverized into powders, or is formed into granules or fine grains, and the outer surfaces of them may be coated with a polymer soluble in a liquid having pH of at least 5.

In this invention, for improving the characteristics and appearance of chitosan, a mixture of chitosan and a powdery cellulose and/or starch, and the polymer soluble in a liquid having pH of at least 5, one or more kinds of additives, a coloring agent, an isotonic agent, and an antioxidant may be added. For examples, additives such as dextrin, mannitol, cyclodextrin, tragacanth gum, etc.; coloring agents such as β-carotene, Red Color No. 2, Blue Color No. 1, etc.; isotonic agents such as sodium chloride, glucose, etc., and antioxidants such as ascorbic acid, erythorbic acid, and the salts and esters thereof can be used.

As described above, when the polypeptide oral medicine is orally administered, the medicine reaches the large intestine without being dissociated, wherein the medicine is dissociated and the polypeptide is effectively adsorbed through the mucous membrane of the large intestine to show the characteristic physiological activity.

Then, the invention is described in more detail by the following examples.

### Preparation Example 1

In an aqueous solution of a mixture of 750 »l of 2% by weight polyoxyethylene (9) octylphenyl ether [Nonidet P-40 (registered trade mark), made by Sigma Co.], 150 »l of an aqueous solution of 1% by weight sodium caprate, and 1500 »l was dissolved 15 mg of human calcitonin to provide an aqueous calcitonin solution. Then, to the solution were added 0.8 g of lactose, 0.3 g of hydroxyethyl cellulose, 3.8 g of a solution obtained by dissolving 10% by weight chitosan having a deacetylation degree of 82 mol% in an aqueous solution of 35% by weight acetic acid, and a small count of a lead powder having a grain size of 74 »m (200 mesh) as a soft X-ray fluoroscopic marker followed by sufficient mixing to provide an original solution for preparing a calcitonin formulation.

The solution was granulated and then vacuum-dried to provide a granular calcitonin formulation having a diameter of about 1 mm. Then, an ethanol solution of 5% by weight Eudragit L (registered trade mark, made by Roehm Pharma Co.) and 1% by weight castor oil was spray coated on the granular formulation to form anti-gastric juice liquid coating of 0.1 g per gram of the formulation and to provide a large intestinal dissociative calcitonin medicine.

The granular calcitonin medicine thus obtained was orally administered to rats (weight: about 250 g) at 200 »g of calcitonin per one rat and the dissociated site of the medicine was inspected by a soft X-ray fluoroscopic inspection during the passage of time. Also, at the same time, the reduction ratios of the calcitonin concentration and the calcium concentration in the serum were measured.

As the results thereof, it was confirmed that the medicine passed through the stomach after 4 hours since the administration and moved to the colon after from 10 to 12 hours but the dissociation of the medicine was scarcely observed before entering the colon, and thereafter, the medicine was dissociated during the time of from 3 to 12 hours. Also, it was confirmed that each of the calcitonin concentration and the calcium concentration in the serum showed the maximum value after 12 hours as shown in Table 1 below, which showed with the above-described inspection result of the dissociated position that calcitonin was most effectively absorbed in the large intestine and at the same time the calcium concentration lowering action by calcitonin was effected.

**Table 1**

| Concentration change of Calcitonin and Calcium in Serum | | | |
|---|---|---|---|
| | Time after Administration (hour) | | |
| | 4 | 6 | 12 |
| Calcitonin Concentration (pg/ml) in Serum | 7 | 13 | 24 |
| Reduction Ratio (%) of Calcium Concentration in Serum | 0.5 | 4.3 | 6.2 |

### Preparation Example 2

After adding 0.5 g of lactose, 0.8 g of corn starch, 0.3 g of hydroxypropyl cellulose, and a small amount of a lead powder to an aqueous calcitonin solution prepared as in Example 1 to form an original solution for preparing the formulation, the solution was subjected to granulation to provide a granular calcitonin formulation. Then, a solution prepared by dissolving 2% by weight chitosan having the deacetylation degree of 86 mol% in an aqueous solution of 0.8% by weight acetic acid was spray coated on the granular formulation to apply a chitosan coating of 0.05 g per gram of the medicament, and further the anti-gastric juice coating was applied as in Preparation Example 1.

When the calcitonin medicine thus obtained was orally administered to rats and the dissociated site was inspected, it was confirmed that the greater part of the medicine was not dissociated before it reached the large intestine and dissociated in the large intestine only.

### Example 1

Molds having the form of a capsule were immersed in a viscous solution formed by dissolving 12% by weight chitosan having a deacetylation degree of 82 mol% in an aqueous solution of 4% by weight acetic acid to form a chitosan solution layer of a constant thickness on the surface of each mold and then by drying the layer with hot air, chitosan capsules having a diameter of about 4.5 mm and a thickness of 0.05 to 0.1 mm were formed.

Then, after preparing capsule medicament by enclosing the granular calcitonin formulation as used in Example 2 in the capsules, the capsules were immersed in a solution formed by dissolving 10 g of Eudragit L and 2 g of castor oil in 90 g of ethanol to form anti-gastric juice coating of about 0.05 mm in thickness on the surfaces of them.

Also, on the other hand, a barium sulfate powder is enclosed in the chitosan capsules and anti-gastric juice coating was applied thereon by the same manner as above.

The calcitonin-enclosed capsules and the barium sulfate-enclosed capsules were simultaneously orally administered to Beagle dogs (weight: about 12 kg). In addition, the dose of calcitonin was 360 »g per one dog. When the dissociated site of the capsules was inspected by a soft X-ray fluoroscopic inspection, it was confirmed that the capsules were not dissociated in the alimentary canal from the stomach to the small intestine, reached the large intestine after 4 to 6 hours since the administration, and dissociated there. Also, as to the calcitonin concentration in the serum, a sudden increase was observed in proportion to the dissociated time of the capsules as shown in Table 2 below.

**Table 2**

| Chance of Calcitonin Concentration in Serum | | | | | | |
|---|---|---|---|---|---|---|
| Time (hour) After Administration | 0 | 2 | 4 | 6 | 8 | 10 |
| Calcitonin Concentration (pg/ml) | 51 | 44 | 70 | 286 | 180 | 47 |

### Example 2

From an original solution obtained by uniformly dispersing 4% by weight powdery cellulose having a grain size of 37 »m (400 mesh) in a solution formed by dissolving 4% by weight chitosan having the deacetylation degree of 82 mol% in an aqueous solution of 12% by weight acetic acid, capsules were formed an in Example 1. In this case, since the concentration of the original solution of about twice higher than the case of using chitosan only, the moldability was improved.

Then, after enclosing the granular calcitonin formulation as used in Preparation Example 2 and a barium sulfate powder in the capsules to provide a capsule medicament, the capsules were immersed in a solution formed by dissolving 10 g of Eudragit L and 2 g of castor oil in 90 g of ethanol to form anti-gastric juice coating of about 0.05 mm in thickness on the surfaces thereof.

The capsule medicine thus obtained was orally administered to 6 Beagle dogs of a group in a fasting state and in a fed state with 3 capsules per dog and the dissociated site of the capsule medicine was inspected by a soft X-ray fluoroscopic inspection with the passage of time.

As the result thereof, it was confirmed that the time for the capsule medicine reaching the large intestine largely differed from 2 hours to 6 hours according to each individual but in each case, the medicine was not dissociated in the alimentary canal before the large intestine and all the formulation was dissociated in the large intestine.

### Example 3

Capsule molds were immersed in a solution formed by uniformly dispersing a starch powder in an aqueous chitosan having the deacetylation degree of 82 mol% in an amount of 70 parts by weight to 100 parts by weight of the chitosan and pulled up therefrom followed by drying with hot air to form capsules having a diameter of 4.5 mm and a thickness of from 0.05 to 0.1 mm. Furthermore, the capsules immersed in a 10% by weight water-containing ethanol solution containing 1% by weight sodium hydroxide for one hour and washed well to provide capsules composed of chitosan and starch.

When the granular calcitonin formulation and a barium sulfate powder were enclosed in each capsule, anti-gastric juice coating applied onto the capsules, the capsule medicine was orally administered to Beagle dogs, and the dissociated position was inspected as in Example 2. It was confirmed that the capsule medicine was not dissociated in the alimentary canal before the large intestine and was dissociated in the large intestine only.

## Claims (Claims for the following Contracting State(s): AT, BE, CH, LI, DE, DK, FR, GB, IT, LU, NL, SE)

1. An oral medicine which is dissociative in the large intestine, comprising a physiologically active polypeptide formulation encapsulated within a molded chitosan hard capsule, the capsule having a surface coated by a layer of a polymer soluble in a liquid of pH5 or higher.

2. An oral medicine which is dissociative in the large intestine comprising a physiologically active polypeptide formulation encapsulated within a hard capsule composed of chitosan and a powdery cellulose or starch, or a mixture of such cellulose and starch, the capsule having a surface coated by a layer of a polymer soluble in a liquid of pH5 or higher.

3. The medicine as claimed in Claims 1 or 2, wherein said polymer soluble in a liquid of pH5 or more is at least one polymer selected from a methacrylic acid-methyl methacrylate copolymer; a methacrylic acid-ethyl acrylate copolymer; hydroxypropylmethyl cellulose acetate succinate: and hydroxypropylmethyl cellulose phthalate.

4. The medicine as claimed in Claim 1, 2 or 3, wherein the viscosity at 20°C of a solution obtained by dissolving the chitosan in an aqueous 1% by weight acetic acid solution at a concentration of 1% by weight is not higher than 100 mPa.s (100 cps) and the degree of deacetylation of chitosan is from 60 mol% to 98 mol%.

5. The medicine as claimed in any of Claims 1 to 4, wherein said hard capsule is produced by forming a layer of a chitosan solution on a surface of a mold having a form for a capsule, and drying said layer to form said capsule.

6. The medicine as claimed in any of Claims 1 to 5, wherein the layer of said capsule has a thickness of from 0.05-0.1 mm.

7. Use of chitosan for the manufacture of the medicine claimed in any one of Claims 1 to 6.

## Claims (Claims for the following Contracting State(s): ES, GR)

1. A method of making an oral medicine which is dissociative in the large intestine, comprising encapsulating a physiologically active polypeptide formulation within a molded chitosan hard capsule, the capsule having a surface coated by a layer of a polymer soluble in a liquid of pH5 or higher.

2. A method of making an oral medicine which is dissociative in the large intestine, comprising encapsulating a physiologically active polypeptide formulation within a hard capsule composed of chitosan and a powdery cellulose or starch, or a mixture of such cellulose and starch, the capsule having a surface coated, by a layer of a polymer soluble in a liquid of pH5 or higher.

3. The method as claimed in Claims 1 or 2, wherein said polymer soluble in a liquid of pH5 or more is at least one polymer selected from a methacrylic acid-methyl methacrylate copolymer; a methacrylic acid-ethyl acrylate copolymer; hydroxypropylmethyl cellulose acetate succinate; and hydroxypropylmethyl cellulose phthalate.

4. The method as claimed in Claim 1, 2 or 3, wherein the viscosity at 20°C of a solution obtained by dissolving the chitosan in an aqueous 1% by weight acetic acid solution at a concentration of 1% by weight is not higher than 100 mPa.s (100 cps) and the degree of deacetylation of chitosan is from 60 mol% to 98 mol%.

5. The method as claimed in any of Claims 1 to 4, comprising an initial step of producing said hard capsule by forming a layer of a chitosan solution on a surface of a mold having a form for a capsule, and drying said layer to form said capsule.

6. The method as claimed in any of Claims 1 to 5, wherein the layer of said capsule is given a thickness of from 0.05-0.1 mm.

7. Use of chitosan for the manufacture of the medicine claimed in any one of Claims 1 to 6.

## Patentansprüche (Patentansprüche für folgende(n) Vertragsstaat(en): AT, BE, CH, LI, DE, DK, FR, GB, IT, LU, NL, SE)

1. Orales Medikament, welches im Dickdarm dissoziierbar ist, das eine physiologisch aktive Polypeptidformulierung enthält, die in einer geformten festen Chitosankapsel eingebettet ist, die Kapsel besitzt eine Oberfläche, die mit einer Schicht aus einem Polymer überzogen ist, dar in einer Flüssigkeit mit einem pH-Wert von pH 5 oder höher löslich ist.

2. Orales Medikament, welches im Dickdarm dissoziierbar ist, das eine physiologisch aktive Polypeptidformulierung enthält, die in einer festen Kapsel eingebettet ist, die aus Chitosan und einer pulverförmigen Cellulose oder Stärke oder einer Mischung einer solchen Cellulose oder Stärke Zusammengesetzt ist, die Kapsel besitzt eine Oberfläche, die mit einer Schicht aus einem Polymer überzogen ist, das in einer Flüssigkeit mit einem pH-Wert von pH 5 oder höher löslich ist.

3. Medikament nach Anspruch 1 oder 2, bei dem das Polymer, das in einer Flüssigkeit mit einem pH-Wert von pH 5 oder mehr löslich ist, wenigstens ein Polymer ist, ausgewählt aus der Gruppe, bestehend aus einem Methacrylsäure-Methylmethacrylatcopolymer; einem Methacrylsäure-Ethylacrylatcopolymer; Hydroxypropylmethylcelluloseacetatsuccinat und Hydroxypropylmethylcellulosephthalat.

4. Medikament nach Anspruch 1, 2 oder 3, bei dem die Viskosität bei 20°C einer Lösung, die durch Lösen von Chitosan in einer wässrigen 1 gewichts-%igen Essigsäurelösung mit einer Konzentration von 1 Gewichts-% erhalten wurde, nicht höher als 100 mPa.s (100 cps) und der Grad der Entacetylierung des Chitosans von 60 Mol-% bis 98 Mol-% beträgt.

5. Medikament nach einem der Ansprüche 1 bis 4, bei dem die feste Kapsel durch Erzeugen einer Schicht einer Chitosanlösung auf der Oberfläche einer Form mit Kapselform und Trocknen dieser Schicht, um diese Kapsel zu bilden, hergestellt wird.

6. Medikament nach einem der Ansprüche 1 bis 5, bei dem die Schicht dieser Kapseln eine Dicke von 0,05 bis 0,1 mm aufweist.

7. Verwendung von Chitosan zur Herstellung des Medikaments nach einem der Ansprüche 1 bis 6.

## Patentansprüche (Patentansprüche für folgende(n) Vertragsstaat(en): ES, GR)

1. Verfahren zur Herstellung eines oralen Medikaments, welches im Dickdarm dissoziierbar ist, das das Einbetten einer physiologisch aktive Polypeptidformulierung in einer geformten festen Chitosankapsel umfaßt, die Kapsel besitzt eine Oberfläche, die mit einer Schicht aus einem Polymer überzogen ist, das in einer Flüssigkeit mit einem pH-Wert von pH 5 oder höher löslich ist.

2. Verfahren zur Herstellung eines oralen Medikaments, welches im Dickdarm dissoziierbar ist, das das Einbetten einer physiologisch aktive Polypeptidformulierung in einer festen Kapsel, die aus Chitosan und einer pulverförmigen Cellulose oder Stärke oder einer Mischung einer solchen Cellulose oder Stärke zusammengesetzt ist, die Kapsel besitzt eine Oberfläche, die mit einer Schicht aus einem Polymer überzogen ist, das in einer Flüssigkeit mit einem pH-Wert von pH 5 oder mehr löslich ist, umfaßt.

3. Verfahren nach Anspruch 1 oder 2, bei dem das Polymer, das in einer Flüssigkeit mit einem pH-Wert von pH 5 oder mehr löslich ist, wenigstens ein Polymer ist, ausgewählt aus der Gruppe, bestehend aus einem Methacrylsäure-Methylmethacrylatcopolymer; einem Methacrylsäure-Ethylacrylatcopolymer; Hydroxypropylmethylcelluloseacetatsuccinat und Hydroxypropylmethylcellulosephthalat.

4. Verfahren nach Anspruch 1, 2 oder 3, bei dem die Viskosität bei 20°C einer Lösung, die durch Lösen von Chitosan in einer wässrigen 1 gewichts-%igen Essigsäurelösung mit einer Konzentration von 1 Gewichts-% erhalten wurde, nicht höher als 100 mPa.s (100 cps) und der Grad der Entacetylierung des Chitosans von 60 Mol-% bis 98 Mol-% beträgt.

5. Verfahren nach einem der Ansprüche 1 bis 4, das einen Anfangsschritt der Herstellung dieser festen Kapsel durch Erzeugen einer Schicht einer Chitosanlösung auf der Oberfläche einer Form mit Kapselform und Trocknen dieser Schicht, um diese Kapsel zu bilden, umfaßt.

6. Verfahren nach einem der Ansprüche 1 bis 5, bei dem die Schicht dieser Kapsel eine Dicke von 0,05 bis 0,1 mm besitzt.

7. Verwendung von Chitosan zur Herstellung des Medikaments nach einem der Ansprüche 1 bis 6.

## Revendications (Revendications pour l'(les) Etat(s) contractant(s) suivant(s): AT, BE, CH, LI, DE, DK, FR, GB, IT, LU, NL, SE)

1. Médicament oral qui est dissociable dans le gros intestin, comprenant une formulation de polypeptide physiologiquement actif encapsulée dans une gélule moulée de chitosan, la gélule ayant une surface revêtue d'une couche d'un polymère soluble dans un liquide de pH 5 ou supérieur.

2. Médicament oral qui est dissociable dans le gros intestin comprenant une formulation de polypeptide physiologiquement actif encapsulée dans une gélule composée de chitosan et de cellulose en poudre ou d'amidon, ou d'un mélange de tels cellulose et amidon, la gélule ayant une surface revêtue d'une couche d'un polymère soluble dans un liquide de pH 5 ou supérieur.

3. Médicament selon la revendication 1 ou 2, dans lequel ledit polymère soluble dans un liquide de pH 5 ou supérieur est au moins un polymère choisi parmi un copolymère de méthacrylate de méthyle-acide méthacrylique; un copolymère d'acrylate d'éthyle-acide méthacrylique; un acétate succinate d'hydroxypropylméthylcellulose; et un phtalate d'hydroxypropylméthylcellulose.

4. Médicament selon la revendication 1, 2 ou 3, dans lequel la viscosité à 20°C d'une solution obtenue en dissolvant le chitosan dans une solution aqueuse d'acide acétique à 1% en poids à une concentration de 1% en poids n'est pas supérieure à 100 mPa.s (100 cps) et le degré de déacétylation du chitosan est compris entre 60% en moles et 90% en moles.

5. Médicament selon l'une quelconque des revendications 1 à 4, dans lequel ladite gélule est produite en formant une couche d'une solution de chitosan sur une surface d'un moule ayant une forme pour une gélule, et en séchant ladite couche pour former ladite gélule.

6. Médicament selon l'une quelconque des revendications 1 à 5, dans lequel la couche de ladite gélule possède une épaisseur de 0,05 à 0,1 mm.

7. Utilisation de chitosan pour la fabrication du médicament selon l'une quelconque des revendications 1 à 6.

## Revendications (Revendications pour l'(les) Etat(s) contractant(s) suivant(s): ES, GR)

1. Procédé pour fabriquer un médicament oral qui est dissociable dans le gros intestin, comprenant l'encapsulation d'une formulation de polypeptide physiologiquement actif dans une gélule moulée de chitosan, la gélule ayant une surface revêtue d'une couche d'un polymère soluble dans un liquide de pH 5 ou supérieur.

2. Procédé pour fabriquer un médicament oral qui est dissociable dans le gros intestin, comprenant l'encapsulation d'une formulation de polypeptide physiologiquement actif dans une gélule composée de chitosan et de cellulose en poudre ou d'amidon, ou d'un mélange de tels cellulose et amidon, la gélule ayant une surface revêtue d'une couche d'un polymère soluble dans un liquide de pH 5 ou supérieur.

3. Procédé selon la revendication 1 ou 2, dans lequel ledit polymère soluble dans un liquide de pH 5 ou supérieur est au moins un polymère choisi parmi un copolymère de méthacrylate de méthyle-acide méthacrylique; un copolymère d'acrylate d'éthyle-acide méthacrylique; un acétate succinate d'hydroxypropylméthylcellulose; et un phtalate d'hydroxypropylméthylcellulose.

4. Procédé selon la revendication 1, 2 ou 3, dans lequel la viscosité à 20°C d'une solution obtenue en dissolvant le chitosan dans une solution aqueuse d'acide acétique à 1% en poids à une concentration de 1% en poids n'est pas supérieure à 100 mPa.s (100 cps) et le degré de déacétylation du chitosan est compris entre 60% en moles et 90% en moles.

5. Procédé selon l'une quelconque des revendications 1 à 4, comprenant une étape initial de production de ladite gélule en formant une couche d'une solution de chitosan sur une surface d'un moule ayant une forme pour une gélule, et en séchant ladite couche pour former ladite gélule.

6. Procédé selon l'une quelconque des revendications 1 à 5, dans lequel on donne à la couche de ladite gélule une épaisseur de 0,05 à 0,1 mm.

7. Utilisation de chitosan pour la fabrication du médicament selon l'une quelconque des revendications 1 à 6.
